# EUROPEAN PATENT APPLICATION

(11) **EP 4 321 154 A1**
(43) Date of publication of application: **14.02.2024**
(21) Application number: 23188856.1
(22) Date of filing: 01.08.2023
(51) Int. Cl.: A61K 9/20, A61K 31/55, A61K 9/16

(54) **A TABLET OF TOLVAPTAN AND AT LEAST ONE BINDER PROCESSED WITH SPRAY GRANULATION**

(30) Priority: 03.08.2022 TR 202212261
(71) Applicant: SANOVEL ILAC SANAYI VE TICARET A.S., Istanbul 34460 (TR)
(72) Inventor: OZDEN, Aydan, Istanbul (TR); KIVANC, Furkan, Istanbul (TR); PEHLIVAN AKALIN, Nur, Istanbul (TR); SUNEL, Fatih, Istanbul (TR)
(74) Representative: Sevinç, Erkan

(57) **Abstract**

The present invention relates to a tablet comprising an intragranular composition comprising tolvaptan and at least one binder, wherein the intragranular composition is obtained by spray granulation with a solution comprising water and binder.

## Description

### Field of the Invention

The present invention relates to a tablet comprising an intragranular composition comprising tolvaptan and at least one binder, wherein the intragranular composition is obtained by spray granulation with a solution comprising water and binder

### Background of the Invention

Tolvaptan is an orally bioavailable, selective, arginine vasopressin receptor 2 (V2, AVPR2) antagonist that can be used to treat hyponatremia. Upon oral administration, tolvaptan selectively and competitively binds to and blocks the V2 receptor located in the walls of the vasculature and luminal membranes of renal collecting ducts, thereby preventing the binding of vasopressin to the V2 receptor. This prevents water absorption in the kidneys and increases the excretion of electrolyte-free water via the kidneys. This reduces intravascular volume and increases serum sodium concentrations and osmolality.

Tolvaptan is chemically described as N-(4-(7-Chloro-5-hydroxy-2,3,4,5-tetrahydro-1H-benzo[b]azepine-1-carbonyl)-3-methylphenyl)-2-methylbenzamide. Its empirical formula is C₂₆H₂₅ClN₂O₃, with structural formula as follows:

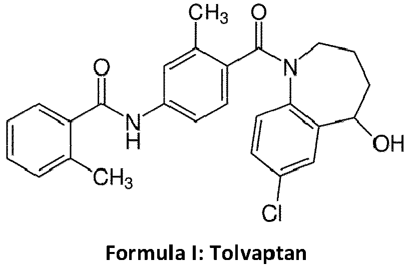

Tolvaptan is available as oral tablets containing 7.5mg, 15 mg and 30 mg of tolvaptan, with trade name SAMSCA^{®} by Otsuka America Pharmaceutical for the treatment of clinically significant hypervolemic and euvolemic hyponatremia.

Tolvaptan is a class IV drug in the BCS classification, that is, a low-soluble and hypotonic drug. For poorly soluble drugs, its dissolution is the rate-limiting process of absorption and is often the most important factor affecting its bioavailability U.S. Pat. No. 5,258,510 disclose tolvaptan.

CN102366412 (A) discloses a preparation method of a tolvaptan tablet. The method is wet granulation without water.

WO2014068586 (A2) discloses solid dispersion compositions of tolvaptan and process for the preparation of oral dosage forms. Solid dispersion is prepared by techniques including top spray granulation. Tablet compositions of Tolvaptan prepared by fluid bed top spray granulation method is mentioned in this patent.

WO2008156217 (A2) discloses a pharmaceutical solid preparation comprising tolvaptan and hydroxypropylcellulose containing a hydroxypropoxyl group in an amount of 50 wt.% or greater. Also, a method for preparing the pharmaceutical solid preparation discloses. The method is wet or dry granulation.

In prior art, there are also several patents which disclose tolvaptan in oral pharmaceutical dosage forms. However, despite the dissolution problem of tolvaptan, an effective formulation and spray granulation (fluized bed granulation) method has not been disclosed.

In terms of the preparation technique, in the preparation of solid dispersions, the active ingredient and the carrier material are dissolved in a solvent and the solvent is evaporated. In solid dispersions, the active ingredient is contained in the solvent. However, a spray granulation method without solid dispersion prepared in this invention has been a good alternative to other solid dispersion composition

There still remains a need in the art to provide an improved a tablet comprising tolvaptan having high solubility, excellent pharmacomechanic properties and accordingly a high bioavailability and a long-term stability which is also obtained by using spray granulation.

### Detailed Description of the Invention

The main object of the present invention is to provide a tablet comprising tolvaptan with having desired level of dissolution rate and high stability and excellent pharmacomechanic properties, such as flowability, compressibility and content uniformity which overcomes the above-described problems in the prior art and have additive advantages over them.

Another object of the present invention is to provides a process for a tablet composition comprising tolvaptan or crystalline polymorph thereof. The process is a simple, rapid, cost effective, time-saving and industrially convenient method.

The term "Tolvaptan" as used herein refers to tolvaptan in the form of the free base or in the form of pharmaceutically acceptable salts, crystalline polymorph, solvates, hydrates, esters or mixture thereof.

Tolvaptan is poorly soluble in water. This causes problems of dissolution profile and bioavailability. In the present invention, some studies have been done so that these problems do not appear.

In addition to solubility in the pharmaceutical composition, stability is also very important. In the present invention, providing a stable formulation and getting to desired bioavailability is achieved with the spray granulation method.

As used herein, the term "intragranular composition" refers to a population of granules that are wetted with solvent and then dried and granulated. Intragranular composition can be called also "granulate" or "granulate component". The "extragranular composition" refers is the part that does not participate in spray granulation.

According to one embodiment of the present invention, a tablet comprising an intragranular composition comprising tolvaptan and at least one binder, wherein the intragranular composition is obtained by spray granulation with a solution comprising water and binder. These properties provide the desired bioavailability and stability.

Suitable binders are selected from the group comprising low substituted hydroxypropylcellulose, polyvinylpyrrolidone, sodium carboxymethyl cellulose, sodium carboxymethyl cellulose, polyethylene glycol, starch, pregelatinized starch, sodium alginate, hydroxypropyl methyl cellulose, hydroxypropyl cellulose, carboxy methyl cellulose, methyl cellulose, polymethacrylates, methacrylate polymers, hyaluronic acid, pectin, polysaccharides, polyacrylamide, polyoxyethylene-alkyl ether, polydextrose, polyethylene oxide or mixtures thereof.

According to one embodiment of the present invention, the binder is low substituted hydroxypropylcellulose.

According to one embodiment of the present invention, low substituted hydroxypropylcellulose contains less than 49% hydropropoxyl group. This ratio provides the desired bioavailability and the desired dissolution profile.

We have found that also low substituted hydroxypropylcellulose having the following particle size is important for formulation. It affects the dissolution properties of the pharmaceutical composition and dosage form and thus the bioavailability. Desired dissolution profile and bioavailability are achieved within the following preferred ranges of the particle size distribution of low substituted hydroxypropylcellulose.

As used here in, "particle size distribution" is defined by the cumulative volume size distribution as tested by a conventionally accepted method which is the laser diffraction method determined by the equipment of Malvern Mastersizer 2000 laser diffraction particle size analyzer analyzer. "D (0.9)" or "d90" means that the size at which %90 by volume of the particles are finer.

According to one embodiment of this invention, low substituted hydroxypropylcellulose average particle size should be in the range of 25 µm to 50 µm and a d(0.9) value should be in the range of 70 µm to 175 µm.

One can determine the average particle size of a solid from a knowledge of its surface area per unit weight and its density; for if the particles are considered to be uniform spheres, then the ratio of volume to area is r/3, where r is the radius of the equivalent sphere.

According to one embodiment of this invention, the amount of binder is between 1.0% and 20.0%, between 1.0% and 15.0% by weight of the total composition.

According to one embodiment of this invention, the amount of binder is between 3.0% and 10.0% by weight of the total composition.

According to one embodiment of this invention, the amount of tolvaptan is between 5.0% and 25.0%, between 8.0% and 25.0%, between 10.0% and 20.0% by weight of the total composition.

According to one embodiment of the present invention, the intragranular composition further comprises at least one pharmaceutically acceptable excipient selected from the group comprising disintegrants, fillers or mixtures thereof.

Suitable disintegrants are selected from the group comprising sodium starch glycolate, croscarmellose sodium, corn starch, starch, low-substituted hydroxypropyl cellulose, carboxymethyl cellulose, sodium carboxymethyl cellulose, calcium carboxymethyl cellulose, sodium carboxymethyl starch, hydroxymethyl starch or mixtures thereof.

According to one embodiment of the present invention, the disintegrant is sodium starch glycolate.

Sodium starch glycolate absorbs water rapidly. This causes swelling leading to rapid disintegration of tablets and granules. Without a disintegrant, tablets may not dissolve properly and may affect the amount of active ingredient, which is absorbed, thus reducing effectiveness. At this point, good solubility and bioavailability have been obtained from the use of sodium starch glycolate which is a superdisintegrant in the formulation.

According to one embodiment of this invention, the amount of disintegrant is between 1.0% and 20.0%, preferably between 3.0% and 15.0%, more preferably between 3.0% and 10.0% by weight of the total composition.

Suitable fillers are selected from the group comprising microcrystalline cellulose, lactose monohydrate, ammonium alginate, calcium carbonate, calcium phosphate, calcium phosphate dehydrate, neutral pellets, calcium sulfate, cellulose, cellulose acetate, compressible sugar, ethylcellulose, fructose, glyceryl palmitostearate, lactitol, lactose, mannitol, magnesium carbonate, magnesium oxide, maltodextrin, maltose, medium chain triglycerides, polydextrose, polymethacrylates, simethicone, sodium alginate, sodium chloride, sorbitol, sucrose, sugar spheres, sulfobutylether beta-cyclodextrin, talc, tragacanth, trehalose, xylitol or mixtures thereof.

According to one embodiment of the present invention, the filler is microcrystalline cellulose or lactose monohydrate or mixtures thereof.

According to one embodiment of this invention, the amount of filler is between 55.0% and 90.0%, between 60.0% and 80.0% by weight of the total composition.

According to one embodiment of this invention, the tablet further comprises an extragranular composition comprising at least one pharmaceutically acceptable excipient selected from the group comprising coloring agents, lubricants, or mixtures thereof.

Suitable coloring agents are selected from the group comprising indigo carmin aliminium lake, ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

According to one embodiment of the present invention, the coloring agent is indigo carmin aliminium lake. This excipient is known as redox indicator. It prevents the active substance from being degraded by giving its own reversible reaction against oxidative and reducing substances (stress factors). Since the reaction is reversible, it is not completely depleted by its own degradation, and the raw material is protected from stress factors (oxidative and reducing species). This contributes positively to the shelf life of the product. This coloring agent provides the desired stability.

Suitable lubricants are selected from the group comprising sodium stearyl fumarate, magnesium stearate, calcium stearate, potassium stearate, stearic acid, sodium chloride, sodium benzoate, sodium acetate, sodium oleate, polyethylene glycols or mixtures thereof.

According to one embodiment of the present invention, the lubricant is sodium stearyl fumarate.

Sodium stearyl fumarate has an effect on tablet strength and disintegration time. At this point, a good flowability was obtained in the compression phase with sodium stearyl fumarate used in the formulation and it was facilitated to provide the desired compression properties. The tablet takes better hardness and sodium stearyl fumarate provides the desired stability to the tablet throughout its shelf life.

According to one embodiment of the present invention, the tablet comprises:
- Tolvaptan
- Low Substituted Hydroxypropylcellulose
- Sodium Starch Glycolate
- Microcrystalline cellulose
- Indigo carmin aliminium lake
- Sodium Stearyl Fumarate
- Lactose Monohydrate

According to this embodiment of the invention, a process for preparing a tablet comprises the following steps:
a) Dissolving Low Substituted Hydroxypropylcellulose in water,
b) Mixing tolvaptan, at least one disintegrant and at least one filler,
c) The mixture prepared is transferred to a fluid bed for spray granulation and low substituted hydroxypropylcellulose:water solution is added to the powder mixture in the fluid bed prepared by the spray method and obtain granule,
d) After the spraying process is finished, the granule is dried until to get the desired humidity value,
e) Milling the dried granule,
f) Adding indigo carmin aliminium lake and then mixing,
g) Adding at least one lubricant and then mixing,
h) Compressing the mixture into tablets.

According to this embodiment of the invention, a process for preparing a tablet comprises the following steps:
a) Dissolving Low substituted hydroxypropylcellulose in water,
b) Mixing tolvaptan, lactose monohydrate, sodium starch glycolate and microcystralline cellulose,
c) The mixture prepared is transferred to a fluid bed for spray granulation and low substituted hydroxypropylcellulose:water solution is added to the powder mixture in the fluid bed prepared by the spray method and obtain granule,
d) After the spraying process is finished, the granule is dried until to get the desired humidity value,
e) Milling the dried granule,
f) Adding indigo carmin aliminium lake and then mixing,
g) Adding sodium stearyl fumarate and then mixing,
h) Compressing the mixture into tablets.

### Example 1:

| | **Amount (% by weight of the total composition)** |
|---|---|
| Tolvaptan | 5.0 - 25.0 |
| Low Subsituted Hydroxypropylcellulose | 1.0 - 20.0 |
| Sodium Starch Glycolate | 1.0 - 20.0 |
| Lactose Monohydrate | 30.0 - 50.0 |
| Microcrystalline cellulose | 25.0 - 40.0 |
| Sodium Stearyl Fumarate | 0.5 - 4.0 |
| Indigo carmin aliminium lake | 0.05 - 1.0 |
| Water* | - |
| **TOTAL** | **100** |

### Example 2:

| | **Amount (% by weight of the total composition)** |
|---|---|
| Tolvaptan | 15.0 |
| Low Substituted Hydroxypropylcellulose | 5.0 |
| Sodium Starch Glycolate | 5.0 |
| Indigo Carmin Aliminium Lake | 0.1 |
| Lactose Monohydrate | 37.0 |
| Microcrystalline cellulose | 36.9 |
| Sodium Stearyl Fumarate | 1.0 |
| Water* | - |
| **TOTAL** | **100** |

**A process for example 1 or 2;**
a) Dissolving low substituted hydroxypropylcellulose in water,
b) Mixing tolvaptan, sodium starch glycolate, lactose monohydrate and microcystralline cellulose,
c) The mixture prepared is transferred to a fluid bed for spray granulation and Low -substituted Hydroxypropylcellulose:water solution is added to the powder mixture in the fluid bed prepared by the spray method and obtain granule.
d) After the spraying process is finished, the granule is dried until to get the desired humidity value(max 3% humidity value)
e) Milling the dried granule,
f) Adding indigo carmin aliminium lake and then mixing,
g) Adding sodium stearyl fumarate and then mixing,
h) Compressing the mixture into tablets.

According to one embodiment of the present invention, the powder mixture which is prepared in step b above, contains the active substance and other excipients. Thus, there is no need for solid dispersion preparation.

According to one embodiment of the present invention, spray granulation (fluid bed granulation) process provides to get more homogeneous powder, adaption easily when equipments and excipients change, good solubility to obtain a desired dissolution properties, improving the compressibility of granules, simple preparation process are in favor of industrial production.

## Claims

1. A tablet comprising an intragranular composition comprising tolvaptan and at least one binder, wherein the intragranular composition is obtained by spray granulation with a solution comprising water and binder.

2. The tablet according to claim 1, wherein binders are selected from the group comprising low substituted hydroxypropylcellulose, polyvinylpyrrolidone, sodium carboxymethyl cellulose, sodium carboxymethyl cellulose, polyethylene glycol, starch, pregelatinized starch, sodium alginate, hydroxypropyl methyl cellulose, hydroxypropyl cellulose, carboxy methyl cellulose, methyl cellulose, polymethacrylates, methacrylate polymers, hyaluronic acid, pectin, polysaccharides, polyacrylamide, polyoxyethylene-alkyl ether, polydextrose, polyethylene oxide or mixtures thereof.

3. The tablet according to claim 2, wherein the binder is low substituted hydroxypropylcellulose

4. The tablet according to claim 1, wherein an intragranular composition further comprises at least one pharmaceutically acceptable excipient selected from the group comprising disintegrants, fillers or mixtures thereof.

5. The tablet according to claim 4, wherein disintegrants are selected from the group comprising sodium starch glycolate, corn starch, starch, low-substituted hydroxypropyl cellulose, carboxymethyl cellulose, sodium carboxymethyl cellulose, calcium carboxymethyl cellulose, sodium carboxymethyl starch, hydroxymethyl starch or mixtures thereof.

6. The tablet according to claim 4, wherein fillers are selected from the group comprising microcrystalline cellulose, lactose monohydrate, ammonium alginate, calcium carbonate, calcium phosphate, calcium phosphate dehydrate, neutral pellets, calcium sulfate, cellulose, cellulose acetate, compressible sugar, ethylcellulose, fructose, glyceryl palmitostearate, lactitol, lactose, mannitol, magnesium carbonate, magnesium oxide, maltodextrin, maltose, medium chain triglycerides, polydextrose, polymethacrylates, simethicone, sodium alginate, sodium chloride, sorbitol, sucrose, sugar spheres, sulfobutylether beta-cyclodextrin, talc, tragacanth, trehalose, xylitol or mixtures thereof.

7. The tablet according to claim 6, wherein the filler is microcrystalline cellulose or lactose monohydrate or mixtures thereof.

8. The tablet according to claim 1, wherein further comprises an extragranular composition comprising at least one pharmaceutically acceptable excipient selected from the group comprising coloring agents, lubricants or mixtures thereof.

9. The tablet according to claim 8, wherein the coloring agent is indigo carmin aliminium lake.

10. The tablet according to claim 8, wherein lubricants are selected from the group comprising sodium stearyl fumarate, magnesium stearate, calcium stearate, , potassium stearate, stearic acid, sodium chloride, sodium benzoate, sodium acetate, sodium oleate, polyethylene glycols or mixtures thereof.

11. The tablet according to claim 10, wherein the lubricant is sodium stearyl fumarate.

12. The tablet according to claim 1, wherein the tablet comprises;
- Tolvaptan
- Low substituted Hydroxypropylcellulose
- Sodium Starch Glycolate
- Microcrystalline cellulose
- Indigo carmin aliminium lake
- Sodium Stearyl Fumarate
- Lactose Monohydrate

13. A process for preparing a tablet comprises the following steps:
a) Dissolving low subtitude hydroxypropylcellulose in water,
b) Mixing tolvaptan, sodium starch glycolate, lactose monohydrate and microcystralline cellulose,
c) The mixture prepared is transferred to a fluid bed for spray granulation and Low subtitude Hydroxypropylcellulose:water solution is added to the powder mixture in the fluid bed prepared by the spray method and obtain granule.
d) After the spraying process is finished, the granule is dried until to get the desired humidity value,
e) Milling the dried granule,
f) Adding indigo carmin aliminium lake and then mixing,
g) Adding sodium stearyl fumarate and then mixing,
h) Compressing the mixture into tablets.
